# EUROPEAN PATENT APPLICATION

(11) **EP 4 640 662 A1**
(43) Date of publication of application: **29.10.2025**
(21) Application number: 24891721.3
(22) Date of filing: 08.11.2024
(51) Int. Cl.: C07C 29/80, C07C 29/04, C07C 31/10, C07C 31/12

(54) **METHOD FOR PRERARING ISOPROPYL ALCOHOL**

(30) Priority: 15.11.2023 KR 20230158482; 25.10.2024 KR 20240147862
(71) Applicant: LG CHEM, LTD., Seoul 07336 (KR)
(72) Inventor: HWANG, Sung June, Daejeon 34122 (KR); LEE, Sung Kyu, Daejeon 34122 (KR); JANG, Kyung Soo, Daejeon 34122 (KR); LEE, Moon Yong, Daejeon 34122 (KR)
(74) Representative: Goddar, Heinz J.
(86) International application number: PCT/KR2024/017595
(87) International publication number: WO 2025/105775

(57) **Abstract**

Provided is a method for preparing isopropyl alcohol, and in the method for preparing isopropyl alcohol, a propylene monomer and water are reacted in a reactor to prepare a reaction product including isopropyl alcohol, all or a part of the reaction product stream is heat exchanged with a side stream of the gas purification column and cooled, and the cooled reaction product is supplied to an absorption tower to reduce energy usage.

## Description

### [Technical Field]

### CROSS-REFERENCE TO RELATED APPLICATIONS

The present application claims the benefit of priorities to Korean Patent Application No. 10-2023-0158482, filed on November 15, 2023, and Korean Patent Application No. 10-2024-0147862, filed on October 25, 2024, the entire contents of which are incorporated herein as a part of the specification.

### Technical Field

The present invention relates to a method of preparing isopropyl alcohol, and more particularly, to a method of separating isopropyl alcohol with high purity from a reaction product from an isopropyl alcohol preparation process and reducing energy costs using waste heat.

### [Background Art]

Isopropyl alcohol (IPA) is used for various purposes including uses as a cleaning agent in the electronic industry such as the manufacture of a semiconductor or a liquid crystal display (LCD).

Isopropyl alcohol is generally prepared by a reaction of a propylene monomer and water, and after the reaction, the reaction product includes isopropyl alcohol, an unreacted propylene monomer, and unreacted water. Herein, isopropyl alcohol is separated and recovered from the reaction product of the isopropyl alcohol preparation process, and the unreacted propylene monomer is recovered and reused in the isopropyl alcohol preparation process.

Meanwhile, when the reaction product is discharged from a reactor into a vapor phase, the gaseous reaction product should be liquefied in order to proceed with a subsequent process such as purification of isopropyl alcohol or recover of the unreacted propylene, and a large amount of waste heat is discarded in this process. Accordingly, there is a need for development of a method for preparing isopropyl alcohol which may reduce process energy by recovering and using discarded waste heat.

### [Disclosure]

### [Technical Problem]

In order to solve the problems mentioned in the Background Art, an object of the present invention is to provide a method of efficiently separating isopropyl alcohol and an unreacted propylene monomer from a reaction product from an isopropyl alcohol preparation process and reducing energy costs used in the process.

### [Technical Solution]

In one general aspect, a method for preparing isopropyl alcohol includes: reacting a propylene monomer and water to prepare a reaction product including propylene and isopropyl alcohol; cooling the reaction product; supplying the cooled reaction product to an absorption tower; introducing a lower discharge stream of the absorption tower including isopropyl alcohol from the absorption tower to an isopropyl alcohol purification part and supplying a part of an upper discharge stream of the absorption tower including propylene to a gas purification column; separating an upper discharge stream of the gas purification column including propylene and a lower discharge stream of the gas purification column including isopropyl alcohol by distillation in the gas purification column and then introducing the lower discharge stream of the gas purification column to the isopropyl alcohol purification part; and obtaining isopropyl alcohol in the isopropyl alcohol purification part, wherein the cooling of the reaction product is performed by including first cooling performed by heat exchange between all or a part of the reaction product stream and a side stream of the gas purification column and second cooling performed by heat exchange between the first cooled reaction product and a refrigerant.

### [Advantageous Effects]

According to the method for preparing isopropyl alcohol of the present invention, a reaction product produced by a reaction of a propylene monomer and water is cooled by heat exchange with a side stream of a gas purification column before supplying the reaction product to an absorption tower, thereby efficiently using thermal energy of the reaction product. Simultaneously, purity of the prepared isopropyl alcohol may be improved.

Specifically, considering absorption efficiency by water in the absorption tower, the high-temperature and gaseous reaction product should be cooled and liquefied in whole or in part, and also be cooled to an appropriate temperature before being introduced to the absorption tower and then supplied to the absorption tower. Meanwhile, since thermal energy is required for separating components by distillation in a gas purification column, the thermal energy of the high-temperature reaction product is supplied to the gas purification column, thereby increasing efficiency of energy use overall.

In particular, since there is a big difference in a temperature profile of the gas purification column between a lower portion and a lower middle portion, a gentle temperature profile between the lower portion and the middle portion of the column needs to be formed by increasing the temperature in the lower middle portion of the column. In the present invention, thermal energy of the high-temperature reaction product is supplied to the side portion of the gas purification column, thereby implementing an overall gentle temperature profile spanning the upper and lower portions of the gas purification column and improving separation performance in the gas purification column. The purity of isopropyl alcohol obtained therefrom may be improved.

### [Description of Drawings]

FIG. 1 is a process flow diagram of the method for preparing isopropyl alcohol according to an exemplary embodiment of the present invention.
FIG. 2 is a process flow diagram of a method for preparing isopropyl alcohol according to the comparative example.
FIG. 3 shows a temperature profile in a column of a conventional gas purification column.
FIG. 4 shows a temperature profile in a column of a gas purification column according to an exemplary embodiment of the present invention.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

Regarding description of the drawings, similar reference numerals may be used for similar or related constituent elements.

A singular form of a noun corresponding to an item may include one or a plurality of items, unless otherwise explicitly stated in the relevant context.

In the present disclosure, each of the phrases such as "A or B", "at least one of A and B", "at least one of A or B", "A, B, or C", "at least one of A, B, and C", and "at least one of A, B, or C" may include any one of the items listed together with the corresponding phrase of the phrases or all possible combinations of them.

The term "and/or" includes a combination of a plurality of described related constituent elements or any one of a plurality of described related constituent elements.

The terms such as "1st" or "2nd", or "first" or "second" may be simply used for distinguishing a corresponding constituent element from other corresponding constituent elements, and the corresponding constituent elements are not limited in other aspects (e.g., importance or order).

In addition, the terms such as "front surface", "back surface", "upper surface", "lower surface", "side surface", "left", "right", "upper", and "lower" used in the present application are defined based on drawings, and the shape and position of each constituent element are not limited by the terms.

The terms "comprises" or "have" are intended to specify the presence of stated features, steps, operations, constituent elements, parts, or a combination thereof, but do not preclude the presence or addition of one or more other features, numerals, steps, operations, constituent elements, parts, or a combination thereof.

When it is described that a constituent element is "connected to", "combined with", "supported on", or "in contact with" other constituent elements, it includes not only the case in which the constituent elements are directly connected, combined, supported, or in contact, but also the case in which they are indirectly connected, combined, supported, or in contact through a third constituent element.

When it is described that a constituent element is positioned "on" other constituent element, it includes not only the case in which the constituent element is in contact with another constituent element, but also the case in which another constituent element is present between the two constituent elements.

The term "stream" used in the present application may refer to a fluid flow in a process, or may refer to a fluid itself flowing in a pipe. Specifically, the stream may refer to both a fluid itself flowing in a pipe connecting each device and a fluid flow. In addition, the fluid may include any one or more components of gas, liquid, and solid.

The term "upper portion" used herein refers to, unless otherwise stated, a height point of 0% to 10% downward from the top of an apparatus, and specifically, may refer to a top (tower top). In addition, the term "lower portion" refers to a height point of 90% to 100% downward from the top of an apparatus, and specifically, may refer to a bottom (tower bottom).

In addition, "pressure" mentioned herein refers to gauge pressure measured under atmospheric pressure conditions.

Meanwhile, unless otherwise particularly stated herein, operation pressure of a column refers to pressure in an upper portion of the column, and operation temperature of a column refers to a temperature in a lower portion of the column.

The present invention relates to a method for preparing isopropyl alcohol (IPA), and hereinafter, the method for preparing isopropyl alcohol of the present invention will be described in detail with reference to the drawings.

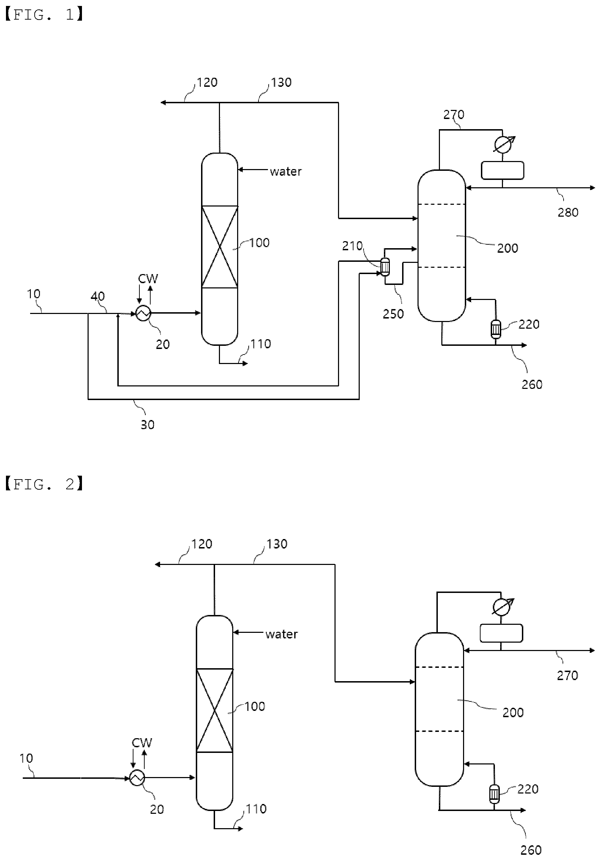

FIG. 1 is a process flow diagram of the method for purifying isopropyl alcohol according to an exemplary embodiment of the present invention.

The method for preparing isopropyl alcohol according to the present invention includes reacting a propylene monomer and water to prepare a reaction product including propylene and isopropyl alcohol.

The isopropyl alcohol may be produced by a process of reacting a propylene monomer and water in a vapor phase. Specifically, a feed stream including a propylene monomer and water is supplied to a reactor and a reaction product produced in the reactor may include isopropyl alcohol, an unreacted propylene monomer, and unreacted water. Here, the isopropyl alcohol is separated from the reaction product and recovered, and the unreacted propylene monomer is recovered and reused in the isopropyl alcohol preparation process.

Specifically, the reactor may be operated under optimal conditions to efficiently prepare isopropyl alcohol by a gaseous reaction of a propylene monomer and water. For example, the operating pressure of the reactor may be 10 kg/cm²·g to 50 kg/cm²·g, 25 kg/cm²·g to 50 kg/cm²·g, or 35 kg/cm²·g to 45 kg/cm²·g, and the operating temperature may be 150°C to 220°C, 165°C to 220°C, or 180°C to 215°C. By operating the reactor in the ranges of pressure and temperature, isopropyl alcohol may be effectively produced by a gaseous reaction using the propylene monomer and water.

The reaction product produced under the operating conditions of the reactor may be a high-temperature and gaseous reaction product including isopropyl alcohol, an unreacted propylene monomer, unreacted water, and inert gas. The reaction product may include 78 to 88 wt% of the (unreacted) propylene and the inert gas with respect to the total mass of the reaction product.

According to an exemplary embodiment of the present invention, in order to supply the gaseous reaction product to an absorption tower 100, first, the reaction product may be cooled.

Some components of the gaseous reaction product are condensed and liquefied by the cooling, and other components are present in the reaction product as a vapor phase and may be supplied to the absorption tower 100. For example, it is preferred that isopropyl alcohol which should be discharged as a lower discharge stream of the absorption tower 100 is present as a liquid phase, and it is preferred that the unreacted propylene, the inert gas, and the like which should be discharged as an upper discharge stream of the absorption tower 100 is present as a vapor phase. That is, efficiency of component separation in the absorption tower may be increased by phase change of some components by the cooling.

Specifically, when isopropyl alcohol is discharged to the upper portion of the absorption tower and fed into the reactor again, it may adversely affect the isopropyl alcohol production reaction performed in the reactor, and thus, it is preferred that isopropyl alcohol is recovered to the lower portion of the absorption tower as much as possible. In addition, when the unreacted propylene and the inert gas is discharged to the lower portion of the absorption tower, an additional gas purification column for recovering the unreacted propylene or the inert gas which have been discharged to the lower portion is needed and the energy usage therefrom is increased, and thus, it is preferred that the unreacted propylene and the inert gas are recovered to the upper portion of the absorption tower as much as possible.

Furthermore, the absorption tower absorbs isopropyl alcohol while absorption water (water) introduced to the upper portion of the absorption tower goes down the absorption tower, and the absorption efficiency of isopropyl alcohol by water in the absorption tower 100 is adjusted to the best temperature range by the cooling, and the reaction product may be supplied to the absorption tower 100. That is, the cooling of the reaction product of the present invention may achieve an effect of increasing the absorption efficiency of isopropyl alcohol in the absorption tower 100 through the phase change of some components of the reaction product and the temperature control of the reaction product.

Referring to FIG. 2 related to the conventional art, the cooling of the reaction product was performed by heat exchange with a refrigerant in at least one or more heat exchanger 20 placed in the front stage of the absorption tower. In this case, waste heat of the high-temperature reaction product may not be used, a large amount of refrigerant required for heat exchange is demanded, and thus, it was difficult to efficiently use energy in many ways.

However, referring to FIG. 1 related to an exemplary embodiment of the present invention, efficient energy use is allowed by heat exchange between the high-temperature reaction product and a side reboiler 210 of a gas purification column in a follow-up process. Specifically, first, thermal energy required for the operation of the gas purification column 200 may be decreased by supplying thermal energy of the high-temperature reaction product to the gas purification column 200. That is, most of the thermal energy required for the operation of the gas purification column 200 described later may be replaced using waste heat. Second, the conventional amount of refrigerant used which is required for cooling of the reaction product may be reduced. Third, in order to increase purification efficiency in the gas purification column 200, it is essential to implement a gentle temperature profile of a column, and a gentle temperature profile spanning the lower portion and the middle portion of the gas purification column may be implemented by supplying the thermal energy of the reaction product to the lower middle portion of the gas purification column.

From this point of view, the temperature of the cooled reaction product may be 90 to 99°C, specifically 90 to 95°C. In this case, absorption efficiency of isopropyl alcohol by water in the absorption tower 100 may be further improved, and simultaneously, unreacted propylene discharged to the lower portion of the absorption tower may be minimized.

Meanwhile, according to an exemplary embodiment of the present invention, the cooling of the reaction product may be performed by including first cooling performed by heat exchange between all or a part of the reaction product stream and a side stream 250 of the gas purification column and second cooling performed by heat exchange between a first cooled reaction product stream and a refrigerant. In general, since heat exchange with the side stream 250 of the gas purification column alone may be not enough to cool the reaction product to the temperature, in this case, additional second cooling by a refrigerant in a heat exchanger 20 may be performed on the first cooled reaction product by the heat exchange with the side stream of the gas purification column. Herein, the refrigerant may be cooling water (CW).

According to an exemplary embodiment of the present invention, the entire reaction product stream 10 may be heat exchanged with the side stream 250 of the gas purification column. In addition, according to another exemplary embodiment of the present invention, as shown in FIG. 1, a part stream 30 of the reaction product stream 10 may be heat exchanged with the side stream 250 of the gas purification column, and the rest stream 40 of the reaction product stream 10 may join the stream which is heat exchanged with the side stream 250 of the gas purification column and be heat exchanged with a refrigerant in the heat exchanger 20. That is, a part of the first cooled reaction product stream joins the rest of the stream of the reaction product to form a joining stream, and the second cooling may be performed by heat exchange between the joining stream and the refrigerant. As described above, the heat exchange between the reaction product and the side stream 250 of the gas purification column has a meaning as a technical means for implementing a gentle temperature profile in the gas purification column 200. Therefore, for appropriate energy supply to the lower middle portion of the gas purification column, not all but a part 30 of the reaction product stream 10 is branched off and participates in the heat exchange with the side stream 250 of the gas purification column.

From this point of view, a ratio of the mass flow rate of the part 30 to the total mass flow rate of the reaction product stream 10 (branching ratio) may be 0.1 to 1, 0.1 to 0.5, specifically 0.1 to 0.4. At the ratio (branching ratio), optimal heat quantity required for the operation of a first reboiler 210 of the gas purification column 200 may be supplied. Herein, the heat quantity required for the operation of the first reboiler 210 refers to energy required for separating unreacted propylene and inert gas to the upper portion and separating isopropyl alcohol and water to the lower portion. Specifically, when the ratio (branching ratio) is less than 0.1, although the gas purification column 200 may receive more heat quantity through the first reboiler 210, an insufficient heat quantity is supplied, and all the heat quantity required for the operation of the first reboiler 210 is not supplied. On the contrary, when the ratio (branching ratio) is excessively high, all the heat quantity required for the operation of the first reboiler 210 may be supplied, but it may not be preferred in terms of equipment cost, in that the capacity and the size of the first reboiler should be larger than necessary for this. Specifically, when the ratio (branching ratio) is more than 0.5, the first reboiler 210 should be larger than necessary, and, in particular, since the reaction product is high-temperature gas, its volume capacity is large, and there is an equipment problem in that piping 30 for receiving it should be excessively large. Therefore, when the ratio (branching ratio) is more than 0.5, the heat quantity which may be transferred to the gas purification column 200 reaches a required value, but since the size of the piping in which a fluid moves is unnecessarily large, it is preferred that the ratio (branching ratio) is maintained at 0.5 or less.

Subsequently, the cooled reaction product may be supplied to the absorption tower 100. Here, the cooled reaction product may be supplied to the absorption tower 100 as a gas-liquid mixed phase. A lower discharge stream of the absorption tower including isopropyl alcohol and an upper discharge stream of the absorption tower including (unreacted) propylene may be separated in the absorption tower 100.

The reaction product may be supplied to the lower end of the absorption tower 100, isopropyl alcohol included in the reaction product is dissolved using a solvent supplied to the absorption tower 100 and separated into the lower portion of the absorption tower 100, and the stream including propylene may be separated into the upper portion. The solvent used in the absorption tower 100 may be, for example, water.

The lower discharge stream 110 of the absorption tower including isopropyl alcohol may be introduced to an isopropyl alcohol purification part. Specifically, the lower discharge stream 110 of the absorption tower includes unreacted water and water as absorption water in addition to isopropyl alcohol, and may include light by-products including diisopropyl ether (DIPE) and heavy by-product including n-propyl alcohol (NPA). Isopropyl alcohol in the isopropyl alcohol purification part may be separated from the light by-products, the heavy by-products, and water and recovered with high purity.

Meanwhile, the upper discharge stream of the absorption tower 100 may include propylene, and more specifically, the upper discharge stream of the absorption tower 100 may include inert gas in addition to propylene, and further include a small amount of isopropyl alcohol, the light by-products, and the heavy by-products. The upper discharge stream of the absorption tower 100 may be discharged from the upper portion of the absorption tower, and a part 130 may be introduced to the gas purification column 200 and the rest 120 may be recycled to a reactor for isopropyl alcohol production. The upper discharge stream of the absorption tower 100 may include 92 to 98 wt% of propylene and inert gas.

According to an exemplary embodiment of the present invention, the operating pressure of the absorption tower 100 may be 20 kg/cm²·g to 40 kg/cm²·g, 25 kg/cm²·g to 40 kg/cm²·g, or 25 kg/cm²·g to 35 kg/cm²·g, and the operating temperature thereof may be 80°C to 110°C, 90°C to 110°C, or 90°C to 100°C. By operating the absorption tower 100 at the pressure and the temperature in the above ranges, the stream may be effectively separated into the upper discharge stream including the unreacted propylene monomer and the lower discharge stream including isopropyl alcohol.

The water used for absorbing isopropyl alcohol in the absorption tower 100 may be supplied to the upper portion of the absorption tower 100. As described above, the water serves to separate isopropyl alcohol and heavy by-products into the lower portion. From this point of view, the mass flow rate (for example, ton/hr) of water supplied to the upper portion of the absorption tower 100 may be 15 to 30% of the total mass flow rate of the reaction product supplied to the absorption tower.

When the mass flow rate ratio of water supplied to the upper portion of the absorption tower 100 is less than 15%, it is difficult for isopropyl alcohol to be sufficiently absorbed in water, and isopropyl alcohol is included in the upper discharge stream of the absorption tower 100. In this case, isopropyl alcohol is included in propylene recycled to the reactor and acts as a factor which inhibits a forward reaction of a synthetic reaction of isopropyl alcohol performed in the reactor. In addition, energy usage for separating isopropyl alcohol in the gas purification column is increased. Meanwhile, when the mass flow rate ratio of water supplied to the upper portion of the absorption tower 100 is more than 30%, even propylene and inert gas which should be discharged as the upper discharge stream of the absorption tower 100 are included in the lower discharge stream of the absorption tower, and thus, an additional gas purification column for separating propylene and inert gas included in the lower discharge stream of the absorption tower is needed and energy usage is increased.

Furthermore, the upper discharge stream of the absorption tower 100 may include 92 to 98 wt% of propylene and inert gas, and in terms of component separation efficiency in the absorption tower, it is preferred that the mass flow rate (for example, ton/hr) of water supplied to the upper portion of the absorption tower 100 is 15 to 30% to the total mass flow rate of the reaction product supplied to the absorption tower.

The method for preparing isopropyl alcohol according to an exemplary embodiment of the present invention may include separating an upper discharge stream 270 of the gas purification column including propylene and a lower discharge stream 260 of the gas purification column including isopropyl alcohol by distillation (200) in the gas purification column 200 and then introducing the lower discharge stream of the gas purification column to the isopropyl alcohol purification part.

Specifically, the upper discharge stream 130 of the absorption tower 100 introduced to the gas purification column 200 may include inert gas in addition to propylene, and though it is a small amount, may include isopropyl alcohol which is not separated yet to the lower portion of the absorption tower 100, water, light by-products of diisopropyl ether, and heavy by-products of n-propyl alcohol. Propylene which is recycled to the reactor may be recovered with higher purity by the gas purification column 200, and isopropyl alcohol which may be lost to the upper portion of the gas purification column 200 may be completely recovered to the lower portion.

As described above, a side stream 250 of the gas purification column 200 may be heat exchanged with all or a part of the reaction product 10 supplied to the absorption tower 100. For this, the gas purification column 200 may include a first reboiler 210 connected to the side portion of the gas purification column 200. That is, the first reboiler may be a side reboiler of the gas purification column 200. That is, the heat exchange between the reaction product 10 and the side stream 250 of the gas purification column may be performed in the first reboiler 210.

More specifically, the side stream 250 of the gas purification column may be discharged at a height point of 55 to 85% downward from the tower top of the gas purification column 200 and heat exchanged with the reaction product. For example, when the side stream 250 is discharged at a height point less than 55%, the section of a contact zone where certain components are vaporized and simultaneously brought into contact with refluxed liquid components, that is, a distillation zone where substantial distillation and purification are performed may be shortened. Meanwhile, when it is discharged at a height point more than 85%, the temperature of the side stream is excessively high, so that heat exchange with the reaction product may be difficult, or an effect by the heat exchange (cooling of the reaction product and heating of the side stream) may not be obtained.

Meanwhile, the side stream of the gas purification column which is heat exchanged in the first reboiler 210 may be resupplied to a stage to which the height point of the gas purification column discharging the side stream belongs.

In addition, a temperature of the side stream discharged from the gas purification column 200 may be 40°C to 80°C or 40°C to 60°C, and a temperature of the side stream which is resupplied to the gas purification column 200 after the heat exchange may be 80°C to 100°C or 85°C to 95°C. Thus, a temperature profile by the height of the gas purification column 200 may be appropriately controlled. Thus, the thermal energy required in the gas purification column, which was conventionally supplied only by the second reboiler 220 may be replaced as much as possible, specifically more than a half.

Meanwhile, according to an exemplary embodiment of the present invention, an operating pressure of the upper portion of the gas purification column 200 may be as high as 15 kg/cm²·g to 20 kg/cm²·g or 16 kg/cm²·g to 19 kg/cm²·g. In addition, the operating temperature of the lower portion of the gas purification column 200 may be 140°C to 180°C or 150°C to 180°C. Within the range of the operating temperature and the operating pressure of the gas purification column 200, separation efficiency of the gas purification column 200 is improved, so that isopropyl alcohol, water, and by-products may be all recovered to the lower portion of the gas purification column 200. Furthermore, inexpensive cooling water may be used as a cooling heat source in the condenser provided in the upper portion of the gas purification column 200.

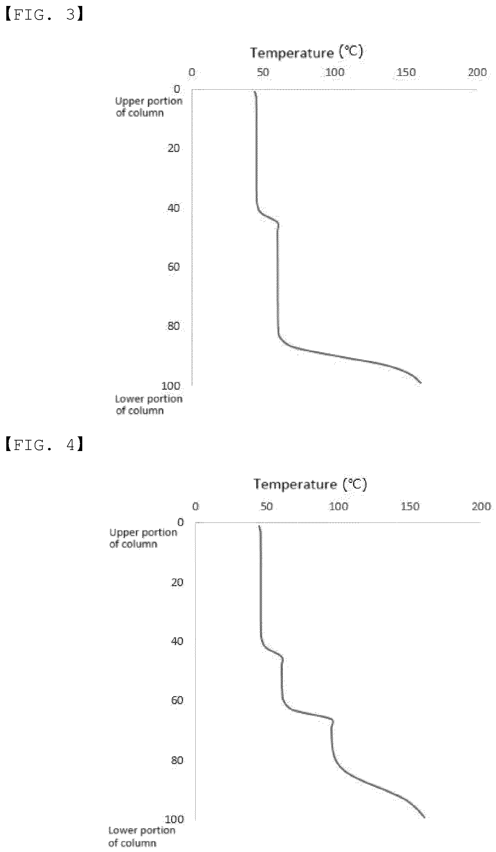

Specifically, FIG. 3 shows a graph showing a temperature profile by height of the gas purification column operated by a common reboiler provided in the lower portion of the gas purification column, without heat exchange between the reaction product and the side stream of the gas purification column. As confirmed in the corresponding temperature profile, since materials having a higher melting point are concentrated in the lower portion of the column, it is recognized that only the lower portion is maintained at a high temperature, and the temperature is rapidly lowered in the lower middle portion and the lowered temperature is maintained to the upper portion. As such, in the temperature section in which the temperature by height remains constant, it is difficult to perform distillation and purification.

However, FIG. 4 shows a graph showing a temperature profile by height of the gas purification column when the reaction product and the side stream 250 of the gas purification column are heat exchanged by the first reboiler 210 according to an exemplary embodiment of the present invention. As a result, it is recognized that a gentle temperature profile of the gas purification column 200 may be implemented, and a section where the temperature by height of the gas purification column changes, that is, a distillation zone where distillation and purification may be performed is increased. That is, the stream introduced to the gas purification column 200 includes heavy components such as isopropyl alcohol and water as well as light components such as propylene and inert gas. That is, since the stream introduced to the gas purification column 200 includes components having a large difference in boiling points, the temperature profile in the gas purification column 200 generally gets steeper as in FIG. 3, and in this case, there is a zone where a temperature change is small and component separation is difficult to be performed depending on the height of the column (so called, dead zone). However, the temperature profile in the gas purification column 200 is gently implemented by the heat exchange by the first reboiler 210 of the present invention, that is, an appropriate temperature gradient depending on the height of the column is implemented, thereby converting the zone where component separation is difficult to be performed into a zone capable of component separation by stripping to increase component separation efficiency of the gas purification column 200.

Also, when the gas purification column 200 is operated only by the second reboiler 220, conventionally as shown in FIG. 2, an advanced heat source (for example, steam) at a high temperature (for example, 160°C or higher) is required for the second reboiler 220, but when the first reboiler 210 is provided in the gas purification column 220 as in an exemplary embodiment of the present invention, the first reboiler 210 may be operated using low level waste heat at about 120°C and, simultaneously, an amount of the advanced heat source used which is required for the second reboiler 220 may be decreased as compared with the conventional technology, and thus, the thermal energy usage may be decreased thereby.

Meanwhile, the gas purification column 200 may further include a second reboiler 220 connected to the lower portion of the gas purification column, in addition to the first reboiler 210. The thermal energy supplied to the gas purification column by the first reboiler may be 50% to 90% to the total thermal energy supplied to the gas purification column by the first reboiler and the second reboiler.

The upper discharge stream 270 of the gas purification column includes propylene and inert gas, and the upper discharge stream 270 of the gas purification column may be supplied to the inert gas removal column through the condenser. In the inert gas removal column, a part of the upper discharge stream of the inert gas removal column including propylene may be recycled to the reactor, and the rest may be purged and discharged out of the system. The gaseous component including the inert gas in the upper discharge stream of the inert gas removal column is purged to separate and remove a part or all of the inert gas, thereby lowering the content of the inert gas in the stream refluxed to the reactor so that the inert gas is not accumulated in the process.

The inert gas may include, for example, one or more selected from the group consisting of hydrocarbons having 2 or 3 carbon atoms, and as a specific example, the inert gas may include one or more selected from the group consisting of ethane and propane.

The lower discharge stream of the absorption tower and the lower discharge stream of the gas purification column include isopropyl alcohol, water, diisopropyl ether (DIPE), and n-propyl alcohol (NPA), and these may pass through an isopropyl alcohol purification part to obtain high-purity isopropyl alcohol.

Hereinafter, the present invention will be described in more detail by the examples. However, the following examples are provided for illustrating the present invention, and it is apparent to a person skilled in the art that various modifications and alterations may be made without departing from the scope and spirit of the present invention, and the scope of the present invention is not limited thereto.

In the following example and comparative example, the method according to the present invention was simulated using a commercial process simulation program, Aspen Plus V12.1.

### Example 1

A process of preparing isopropyl alcohol was performed according to the process diagram as shown in FIG. 1.

Specifically, water and propylene were supplied to a reaction part and reacted in a vapor phase to produce a gaseous reaction product including isopropyl alcohol, water, and propylene. The temperature of a stream 10 including the reaction product was 120°C. Before supplying the stream 10 including the reaction product to an absorption tower 100, the reaction product stream 10 was branched off and a part of the stream was heat exchanged with a first reboiler 210 provided in the side portion of the gas purification column 200. Herein, the mass flow rate of the part of the branched stream was a ratio of 0.2 to the total mass flow rate of the reaction product stream 10.

The gas purification column 200 was operated at an operating pressure of the upper portion of 15 kg/cm²·g and an operating temperature of the lower portion of 160°C. The heat required for the operation of the gas purification column 200 was supplied through the first reboiler 210 connected to the side portion and the second reboiler 220 connected to the lower portion, of the gas purification column 200.

The side stream of the gas purification column 200 was discharged from a height point of 30% downward from the tower top of the gas purification column and heat exchanged with a part of the stream branched from the reaction product stream 10. Herein, the temperature of the side stream discharged from the gas purification column 200 was 60°C, and the temperature of the stream supplied to the gas purification column 200 again after the heat exchange was 95°C.

Meanwhile, a part of the stream branched from the reaction product stream 10 was heat exchanged with the side stream of the gas purification column, joined the remaining stream branched from the reaction product stream 10 at a temperature of 105°C, and introduced to a heat exchanger 20. It was further cooled in the heat exchanger 20 and then supplied to the absorption tower 100, and the temperature of the reaction product at a point when supplied to the absorption tower 100 was 95°C.

The upper discharge stream of the absorption tower 100 included isopropyl alcohol and water, in addition to propylene, and the isopropyl alcohol and water were discharged to the lower portion of the gas purification column 200 and the propylene was discharged to the upper portion of the gas purification column 200.

At this time, the mass flow rate ratio between the upper discharge stream 280 and the lower discharge stream 260 of the gas purification column was 97.7 and 2.3, respectively, when the mass flow rate of the feed 130 of the gas purification column was 100. Meanwhile, the upper discharge stream 280 included 0.03 wt% of ethane, 98.77 wt% of propylene, and 1.2 wt% of propane, the lower discharge stream 260 did not include ethane, propylene, and propane, and inert gas and unreacted propylene were all removed to the upper portion of the gas purification column.

As a result, the thermal energy supplied through the first reboiler and the second reboiler for the operation of the gas purification column 200 was 82 kW and 18 kW, respectively. Meanwhile, refrigerant energy required in the heat exchanger 20 provided in the front stage of the absorption tower for supplying the reaction product at a temperature of 95°C to the absorption tower was 396 kW.

### Comparative Example 1

A process of preparing isopropyl alcohol was performed according to the process diagram as in FIG. 2, for the reaction product having the same component and temperature as Example 1. Furthermore, the operation conditions were controlled so that the flow rates and the compositions of the upper discharge stream and the lower discharge stream of the gas purification column 200 were the same as those of Example 1.

That is, in Comparative Example 1, the gas purification column 200 was not provided with the first reboiler, and provided the same amount of energy (100 kW) as the thermal energy supplied through the first and second reboilers in Example 1, through the second reboiler provided in the lower portion of the gas purification column 200.

In this case, in order to cool the reaction product to 95°C identical to Example 1 as the temperature appropriate for supply to the absorption tower, refrigerant energy of 478 kW needs to be supplied through the heat exchanger 20 provided in the front stage of the absorption tower.

### [Description of symbols]

- 10:: Reaction product
- 100:: Absorption tower
- 200:: Gas purification column
- 210:: First reboiler
- 220:: Second reboiler

## Claims

1. A method for preparing isopropyl alcohol, the method comprising:
reacting a propylene monomer and water to prepare a reaction product including propylene and isopropyl alcohol;
cooling the reaction product;
supplying the cooled reaction product to an absorption tower;
introducing a lower discharge stream of the absorption tower including isopropyl alcohol from the absorption tower to an isopropyl alcohol purification part and supplying a part of an upper discharge stream of the absorption tower including propylene to a gas purification column;
separating an upper discharge stream of the gas purification column including propylene and a lower discharge stream of the gas purification column including isopropyl alcohol by distillation in the gas purification column and then introducing the lower discharge stream of the gas purification column to the isopropyl alcohol purification part; and
obtaining isopropyl alcohol in the isopropyl alcohol purification part,
wherein the cooling of the reaction product is performed by including first cooling performed by heat exchange between all or a part of the reaction product stream and a side stream of the gas purification column and second cooling performed by heat exchange between the first cooled reaction product and a refrigerant.

2. The method for preparing isopropyl alcohol of claim 1,
wherein the side stream of the gas purification column is discharged at a height point of 55 to 85% downward from a tower top of the gas purification column and heat exchanged with the reaction product, and
the heat exchanged side stream of the gas purification column is resupplied to a stage to which the height point of the gas purification column discharging the side stream belongs.

3. The method for preparing isopropyl alcohol of claim 1, wherein a temperature of the side stream discharged from the gas purification column is 40°C to 80°C.

4. The method for preparing isopropyl alcohol of claim 1, wherein a temperature of the cooled reaction product supplied to the absorption tower is 90 to 99°C.

5. The method for preparing isopropyl alcohol of claim 1,
wherein the gas purification column includes a first reboiler connected to a side portion of the gas purification column and a second reboiler connected to a lower portion of the gas purification column, and
the heat exchange between all or a part of the reaction product stream and the side stream of the gas purification column is performed in the first reboiler.

6. The method for preparing isopropyl alcohol of claim 5, wherein the thermal energy supplied to the gas purification column by the first reboiler is 50% to 90% to the total thermal energy supplied to the gas purification column by the first reboiler and the second reboiler.

7. The method for preparing isopropyl alcohol of claim 1,
wherein the first cooling is performed by the heat exchange between a part of the reaction product stream and the side stream of the gas purification column,
a part of the first cooled reaction product stream joins the rest of the reaction product stream to form a joining stream, and
the second cooling is performed by heat exchange between the joining stream and the refrigerant.

8. The method for preparing isopropyl alcohol of claim 7, wherein a ratio of a mass flow rate of the part of the reaction product stream to a total mass flow rate of the reaction product stream is 0.1 to 0.5.

9. The method for preparing isopropyl alcohol of claim 1, wherein the upper portion of the gas purification column is operated at a pressure of 15 kg/cm²·g or more and 20 kg/cm²·g or less.

10. The method for preparing isopropyl alcohol of claim 1, wherein the lower portion of the gas purification column is operated at a temperature of 140°C or higher and 180°C or lower.

11. The method for preparing isopropyl alcohol of claim 1,
wherein the upper discharge stream of the gas purification column includes propylene and inert gas, and
the upper discharge stream of the gas purification column is supplied to an inert gas removal column, a part of an upper discharge stream of the inert gas removal column including propylene is recycled to the reactor, and the rest is purged.

12. The method for preparing isopropyl alcohol of claim 1, wherein the lower discharge stream of the absorption tower and the lower discharge stream of the gas purification column include isopropyl alcohol, water, diisopropyl ether (DIPE), and n-propyl alcohol (NPA).

13. The method for preparing isopropyl alcohol of claim 1,
wherein water is supplied to the upper portion of the absorption tower, and
a mass flow rate of the water supplied to the upper portion of the absorption tower is 15 to 30% to a total mass flow rate of the reaction product supplied to the absorption tower.
